# EUROPEAN PATENT SPECIFICATION

(11) **EP 1 359 149 B1**
(45) Date of publication and mention of the grant of the patent: **31.12.2008**
(21) Application number: 02253146.1
(22) Date of filing: 03.05.2002
(51) Int. Cl.: C07D 487/04, C07D 249/14

(54) **Process for preparing triazolopyrimidine derivatives**
Verfahren zur Herstellung von Triazolopyrimidinderivaten
Procédé de préparation de dérivés de la triazolopyrimidine

(43) Date of publication of application: 05.11.2003
(73) Proprietor: Sinon Corporation, Yang, Wen-Bin, Taichung City (TW)
(72) Inventor: Chen, Chien-Hsing, Taichung City, Taiwan (CN); Yeh, Chun-Lin, Taichung City, Taiwan (CN); Chuang, Yu-Hwa, Taichung City, Taiwan (CN)
(74) Representative: Wakerley, Helen Rachael

(56) References cited:
- JP-A- 62 273 980
- JP-A- 62 273 981
- JP-A- 63 222 171
- US-A- 3 689 488
- US-A- 4 772 720

## Description

### FIELD OF THE INVENTION

This invention relates to a novel process for preparing triazolopyrimidine derivatives of the formula (I): 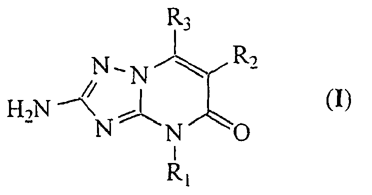 wherein R₁ represents a hydrogen or an alkyl radical of one to ten carbon atoms, or a cycloalkyl radical of three to six carbon atoms, or an alkenyl radical of up to four carbon atoms; R₂ represents a hydrogen, a halogen atoms, a hydroxyalkyl or alkyl radical of one to ten carbon atoms; R₃ represents a hydrogen, a hydroxyalkyl or alkyl radical of one to four carbon atoms.

### BACKGROUND OF THE INVENTION

The compounds of the formula (**I**) are known and described in U.S. Pat. No.3,689,488. They are capable of preventing bronchospasm and therefore useful in the treatment of diseases that involve spasm or constriction of the bronchial muscle, for example asthma or bronchitis. A process for the preparation of the formula (**I**) described in U.S. Pat. No.3,689,488 are summarized as follows:

### (a) Preparation of diamino-1,2,4-triazole (shown in Scheme 1)

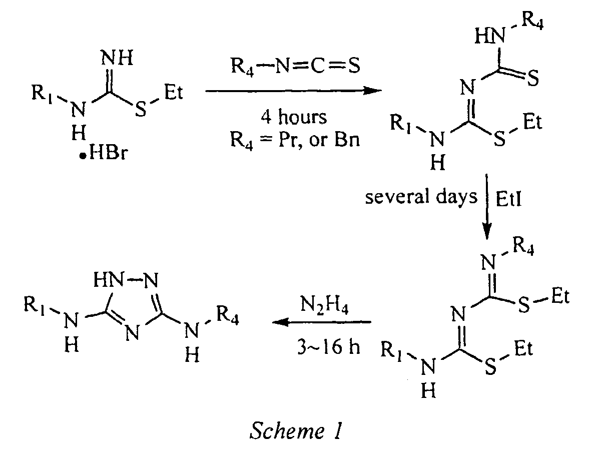

### (b) Preparation of triazolopyrimidines (shown in Scheme 2)

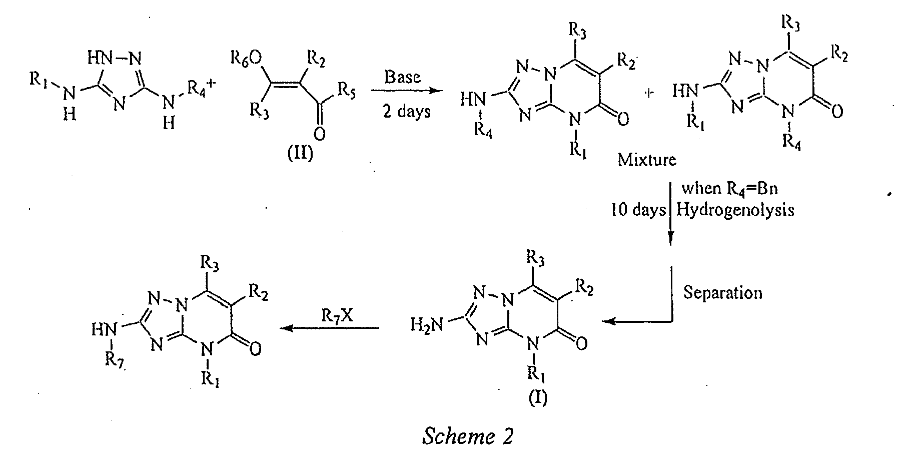

There are several disadvantages for manufacturing in the aforementioned processes. First of all, the process for preparing diamino-1,2,4-triazole (*Scheme 1*) taking several days is inefficient. Second, the condensation of diamino-1,2,4-triazole and α, β-unsaturated acid derivatives of the formula **(II)** that obtained mixture isomers is unselective and need further purification (*Scheme 2*). Third, The hydrogenolysis of benzyl group (deprotection of amino group) to get triazolopyrimidines of the formula **(I)** taking 10 days is time-consuming (*Scheme 2*). From the commercial point of view, the processes shown in scheme 1 and 2 are inefficient and costly.

### SUMMARY OF THE INVENTION

Therefore, it is an object of the present invention to provide a more efficient, cheaper and selective process for preparing triazolopyrimidines derivatives of formula **(I)** that is capable of overcoming the aforesaid drawbacks.
According to the present invention, there is provided a process for preparing triazolopyrimidines derivatives of the formula **(I).** This process is set out in the claims and comprises preparing rapidly diamino-1,2,4-triazole of the formula **(III)** from dialkyl cyanodithioimino carbonate of the formula **(V);** and efficient protection and deprotection of amino group; and selective condensation of α,β -unsaturated acid derivatives of the formula **(II)** and imine of the formula **(VI).**

### DETAILED DESCRIPTION OF THE INVENTION

A process for preparing triazolopyrimidines derivatives of the formula **(I)** according to the present invention comprises four steps. The first step is the efficient preparation of diamino-1,2,4-triazole of the formula **(III),** wherein 1 to 2 equivalent of alkyl amine of the formula **(IV)** is added slowly into the mixture of the dialkyl cyanodithioimino carbonate of the formula (**V**) (1 equivalent) and appropriate solvent at room temperature, and then 1 to 4 equivalent of the hydrazine is added dropwisely after heating to reflux. 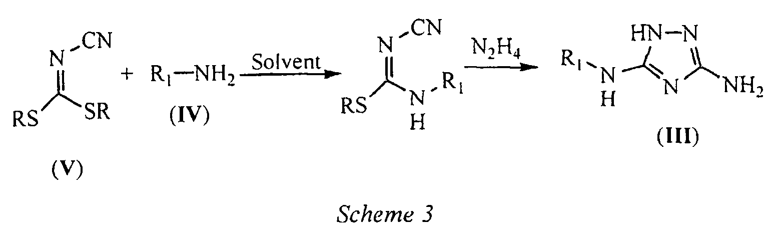 Schematic illustration of preparing diamino-1,2,4-triazole of the formula (**III**) is shown in scheme 3, wherein R₁ has the meaning indicated above; and R represents an alkyl radical of one to four carbon atoms; and solvent can use water, acetonitrile or alcoholic solvent, such as methanol, ethanol, propanol, or butanol, *etc*..

The second step is the formation of imine of the formula (**VI**), wherein 1 to 5 equivalent of aldehyde of the formula (**VII**) is reacted with 1 equivalent ofthe diamino-1,2,4-triazole of the formula (**III**)in an organic solvent under acid catalysis (0 to 1 equivalent) at 50∼150°C. 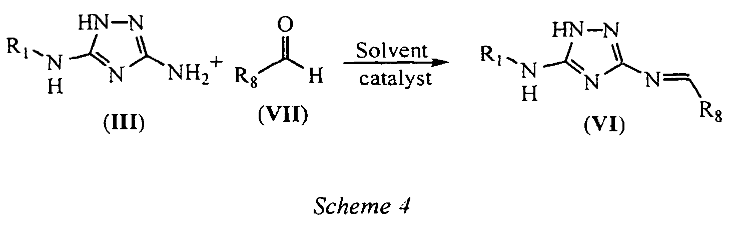 Schematic illustration of preparing imine of the formula (**VI**) is shown in scheme 4, wherein R₁ has the meaning indicated above; and R₈ represents an alkyl radical of one to ten carbon atoms, or a phenyl, or a substituted phenyl; and solvent can use acetonitrile, toluene, xylene, chlorobenzene, chloroform, dichloromethane, ethylene dichloride or alcoholic solvent, such as methanol, ethanol, propanol, or butanol, *etc.*; and catalyst can be not necessary to add, or catalyst can be organic or inorganic acid such as acetic acid, toluene sulfonic acid, sulfuric acid, or hydrogen chloride.

The third step is the condensation of α, β-unsaturated acid derivatives of the formula (**II**) and imine of the formula (**VI**), wherein 1 to 2 equivalent of α, β-unsaturated acid derivatives of the formula (**II**) is reacted with 1 equivalent of the imine of the formula (**VI**) in the present of a base (0.1 to 2 equivalent) and a polymer inhibitor (0.005 to 0.2 equivalent ) in an organic solvent at reflux temperature (40∼150°C). 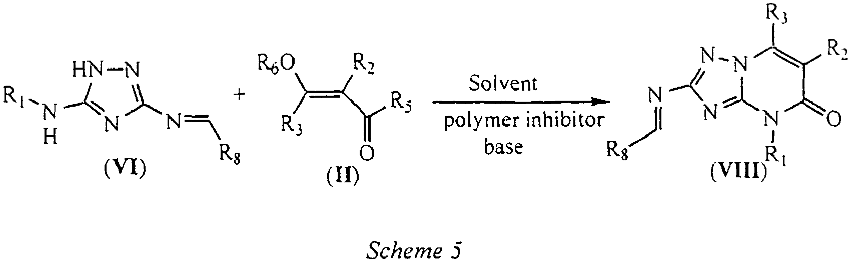 Schematic illustration of the condensation of α, β-unsaturated acid derivatives of the formula (**II**) and imine of the formula (**VI**) is shown in scheme 5, wherein R₁, R₂, R₃ and R₈ have the meaning indicated above, and R; represents a halogen atom or OR₉, wherein R₉ represents an alkyl radical of one to six carbon atoms; and R₆ represents an alkyl radical of one to six carbon atoms; and the solvent can be acetonitrile, toluene, xylene, chlorobenzene, chloroform, dichloromethane or ethylene dichloride; and the base can be metal carbonate or metal hydrogencarbonate, wherein metal can be Li, Na, K, Rb, Cs, Be, Mg, Ca, Sr, or Ba *etc.*; and polymer inhibitor can use hydroquinone or monomethyl ether hydroquinone. An alternative polymer inhibitor is p-methoxyphenol.

The last step is the formation of triazolopyrimidines derivatives of the formula **(I)**, wherein imine of the formula **(VIII)** is hydrolyzed in the present of an acid (0.1 to 10 equivalent), water and solvent at room temperature to refluxing temperature. 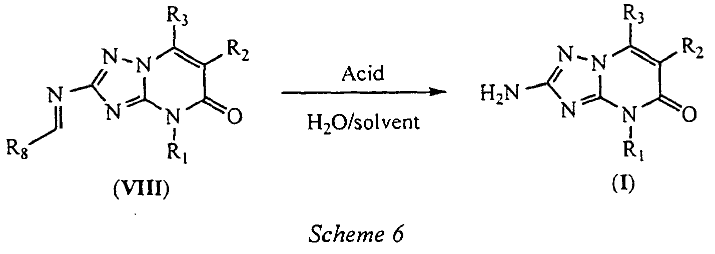 Schematic illustration of the formation of triazolopyrimidines derivatives of the formula (**I**) is shown in scheme 6, wherein R₁, R₂, R₃ and R₈ have the meaning indicated above; and the acid can be inorganic or organic acid such as hydrochloride, sulfuric acid, acetic acid, or oxalic acid, *etc.*; and solvent can use water, methanol, ethanol, acetonitrile, toluene, xylene, chlorobenzene, chloroform, dichloromethane, or ethylene dichloride, *etc..*

The following non-limitative examples further illustrate the present invention.

### EXAMPLE 1

Preparation of N⁵-Propyl-1H-[1,2,4]triazole-3,5-diamine

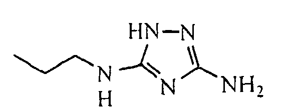

1200 g (8.13 mole) of dimethyl cyanodithioimino carbonate was dissolved in 6 Kg of isopropanol in a 10-liter, four-necked flask equipped with a reflux condenser, thermometer, addition funnel, and mechanical stirrer. 538 g (8.94 mole) of n-propyl amine was added dropwisely over a period of 2 hours at room temperature. After the reaction mixture was stirred for another 20 minutes, the temperature was raised to refluxing temperature and then 1224 g (20.8 mole) of hydrazine monohydrate (85%) was added slowly, followed by reflux for 2∼3 hours. Then 80% of isopropanol was distilled out under reduced pressure. The residue was cooled to 5∼10°C. After filtering and drying, the white solid of N⁵-Propyl-1H- [1, 2, 4] triazole-3, 5-diamine was obtained. The yield is 1075 g (AI 98%, 92% of the theoretical amount). The melting point is 148∼149°C.

### EXAMPLE 2

### Preparation of N³-Benzylidene-N⁵-propyl-1H-[1,2,4]triazole-3,5-diamine

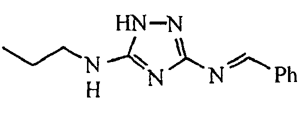

### Method 1

A 5-liter flask equipped with a reflux condenser, thermometer, and mechanical stirrer was charged with 251.8 g (1.75 mole) of N⁵-Propyl-1H- [1,2,4] triazole-3,5-diamine, 1.24 liter of methanol, 194.8 g (1.84 mole) of benzaldehyde and then heated to reflux for 6∼10 hours. After checking the end of the reaction by LC (peak area of N⁵-Propyl-1H-[1,2,4]triazole- 3,5-diamine is less than 1%), 2/3 volume of the solvent was distilled out from the mixture under reduced pressure. The residue was cooled to room temperature and the yellow solid of N³-Benzylidene- N⁵-propyl-1H-[1,2,4]triazole- 3,5-diamine was precipitated. Collecting the precipitate by filtration, 381∼389 g (purity is 98%, 93∼95% of the theoretical amount) of the desired product was obtained after drying.

Similar results were achieved when methanol was replaced by ethanol, toluene or acetonitrile. When 5.2 g (0.087 mole) of acetic acid was used in these cases or/and water was removed azeotropically, the reaction time could be shortened to 1∼4 hours without obvious changing the yield and purity.

### Method 2

A 5-liter flask equipped with a reflux condenser, thermometer, and mechanical stirrer was charged with 251.8 g (1.75 mole) of N⁵-Propyl-1H-[1,2,4]triazole-3,5-diamine, 1.75 liter of toluene, 203.4 g (1.92 mole) of benzaldehyde and 5.2 g (0.087 mole) of acetic acid and then heated to reflux for 1∼3 hours. During the reaction, water was formed as by-product and removed azeotropically. After checking the end of the reaction by LC (peak area of N⁵-Propyl-lH-[1,2,4]triazole- 3,5-diamine is less than 1%), the mixture was cooled down and gave the crude N³-Benzylidene-N⁵-propyl-1H-[1,2,4]triazole-3,5-diamine suspension solution, which was used for the next stage without purification.

### EXAMPLE 3

### Preparation of 2-(Benzylidene-amino)-6-methyl-4-propyl-4H-[1,2,4]triazolo[1,5-a]pyrimidin-5-one

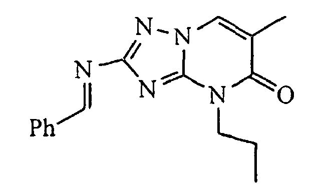

### Method 1

A 5-liter flask equipped with a reflux condenser, thermometer, and mechanical stirrer was charged with 401.03 g (1.75 mole) of N³-Benzylidene-N⁵-propyl-1H-[1,2,4]triazole-3,5-diamine, 253 g (1.83 mole) of potassium carbonate, 250 g (1.92 mole) of methyl 2-methoxy-apha-methyl acrylate, 1.1 g (0.009 mole) of *p*-methoxy phenol, and 1750 ml of acetonitrile and then heated to reflux for 24∼28 hours. After distilling out acetonitrile under reduced pressure, water was added and the mixture was extracted with ethylene dichloride. Ethylene dichloride was removed from extract and then 406∼432 g (77∼82% of the theoretical amount) of the desired product was obtained after drying.

Similar result was achieved when acetonitrile was replaced by toluene.

### Method 2

253 g (1.83 mole) of potassium carbonate, 250 g (1.92 mole) of methyl 2-methoxy-apha-methyl acrylate, and 1.1 g (0.009 mole) of *p*-methoxy phenol were added to the N³-Benzylidene-N⁵-propyl-1H-[1,2,4]triazole-3,5-diamine suspension solution prepared in example 2 (Method 2). After refluxing for 12∼20 hours, the mixture was cooled to room temperature and then 1 liter of water was added. The solid of 2-(Benzylidene-amino)-6-methyl-4-propyl-4H-[1,2,4] triazolo[1,5-a]-pyrimidin-5-one was collected by filtration and the filtrate was separated into two layers which organic layer could be reused for example 2. The crude 2-(Benzylidene-amino)-6-methyl-4-propyl-4H-[1,2,4]triazolo[1,5-a]-pyrimidin-5-one was used for the next stage without purification.

### EXAMPLE 4

### Preparation of 2-Amino-6-methyl-4-propyl-4H-[1,2,4]triazolo[1,5-a]pyrimidin-5-one

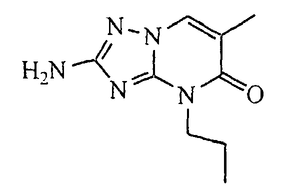

1750 ml of 2N HCl (3.5 mole) was added to the crude 2-(benzylideneamino)-6-methyl-4-propyl-4H-[1,2,4]triazolo[1,5-a]-pyrimidin-5-one prepared in example 3 (Method 2). The mixture was heated to 65∼70°C for 30 minutes and the hydrolyzed benzaldehyde was removed azeotropically under reduced pressure. After benzaldehyde was removed completely, the mixture was cooled down and the pH value was adjusted to 7∼8 with 45% of NaOH_{(aq)}. Then the resulted suspension was filtered and the light yellow solid of 2-amino-6-methyl-4-propyl-4H- [1,2,4]triazolo[1,5-a]pyrimidin-5-one (purity is 98%) was obtained after drying. The yield is 264∼275 g (73 ∼ 76% of the theoretical amount from example 2).

Similar result was achieved when additional 1500 ml of methanol was used as solvent and hydrolyzed at room temperature.

## Claims

1. A process for preparation of triazolopyrimidine derivatives of the formula (I) 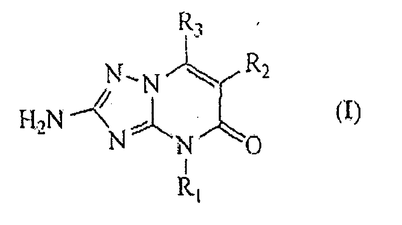 wherein
R₁ represents a hydrogen or an alkyl radical of one to ten carbon atoms or a cycloalkyl radical of three to six carbon atoms, or an alkenyl radical of up to four carbon atoms;
R₂ represents a hydrogen, a halogen atoms, a hydroxyalkyl or alkyl radical of one to ten carbon atoms;
R₃ represents a hydrogen, a hydroxyalkyl or alkyl radical of one to four carbon atoms;
comprises the steps of
(a) reacting dialkyl cyanodithioimino carbonate of the formula **(V),** wherein R represents an alkyl radical of one to four carbon atoms, with alkyl amine of formula **(IV),** wherein R₁ has the meaning as indicated above, in the presence of an appropriate solvent at room temperature, and then adding hydrazine dropwisely after heating to reflux, to give a compound of diamino-1,2,4-triazole of the formula **(III),** wherein R₁ has the meaning as indicated above;
(b) reacting the compound of the formula **(III),** wherein R₁ has the meaning as indicated above, with aldehyde of the formula (VII), wherein R₈ represents an alkyl radical of one to ten carbon atoms, or a phenyl, or a substituted phenyl, in the presence of an organic solvent, to give a compound of imine of the formula **(VI),** wherein R₁ and R₈ have the meanings as indicated above, and wherein 1 to 5 equivalent of aldehyde of the formula **(VII)** is reacted with 1 equivalent of the diamino-1,2,4-triazole of the formula **(III)** in an organic solvent at 50~150 °C;
(c) formation of imine of the formula **(VIII),** wherein R₁, R₂, R₃ and R₈ have the meanings as indicated above, with selective condensation of *α,β*-unsaturated acid derivatives of formula **(II),** wherein R₂ and R₃ have the meanings as indicated above, and R₅ represents a halogen atom or OR₉, wherein R₉ represents an alkyl radical of one to six carbon atoms; and R₆ represents an alkyl radical of one to six carbon atoms, and imine of formula **(VI),** wherein R₁ and R₈ have the meanings as indicated above, in the presence of a base and a polymer inhibitor in an organic solvent at reflux temperature; and
(d) formation of triazolopyrimidine derivatives of formula (I) from the compound of the formula (VIII), wherein R₁, R₂, R₃ and R₈ have the meanings as indicated above, in the present of an acid at room temperature to refluxing temperature.

2. A process according to claim 1, wherein the alkyl amine of the formula (IV) is added slowly into the mixture of the dialkyl cyanodithioimino carbonate of the formula (V) at room temperature and then hydrazine is added dropwisely after heating to reflux, to give a compound of diamino-1,2,4-triazole of the formula (III), in the step (a).

3. A process according to claim 1, wherein the alkyl amine is used 1 to 2 equivalent.

4. A process according to claim 1, wherein the dialkyl cyanodithioimino is used 1 equivalent.

5. A process according to claim 1, wherein the solvent in the step (a) is water, acetonitrile or alcoholic solvent.

6. A process according to claim 5, wherein the alcoholic solvent is methanol, ethanol, propanol or butanol.

7. A process according to claim 1, wherein the hydrazine is used 1 to 4 equivalent.

8. A process according to claim 1, wherein the step (b) occurs under acid catalysis.

9. A process according to claim 8, wherein the acid catalyst is an organic or inorganic acid.

10. A process according to claim 9, wherein the acid catalyst is acetic acid, toluene sulfonic acid, sulfuric acid, or hydrogen chloride.

11. A process according to claim 1, wherein the solvent in the step (b) is acetonitrile, toluene, xylene, chlorobenzene, chloroform, dichloromethane, ethylene dichloride or alcoholic solvent.

12. A process according to claim 11, wherein the alcoholic solvent is methanol, ethanol, propanol or butanol.

13. A process according to claim 1, wherein 1 to 2 equivalent of α,β -unsaturated acid derivatives of the formula (II) is reacted with 1 equivalent of the imine of the formula **(VI)** at 40∼150 °C in the step (c).

14. A process according to claim 1, wherein the base in the step (c) is used 0.1 to 2 equivalent.

15. A process according to claim 1, wherein the polymer inhibitor in the step (c) is used 0.005 to 0.2 equivalent.

16. A process according to claim 1, wherein the solvent in the step (c) is acetonitrile, toluene, xylene, chlorobenzene, chloroform, dichloromethane or ethylene dichloride.

17. A process according to claim 1, wherein the base in the step (c) is a metal carbonate or metal hydrogencarbonate.

18. A process according to claim 17, wherein the metal is Li, Na, K, Rb, Cs, Be, Mg, Ca, Sr, or Ba.

19. A process according to claim 17, wherein the base is potassium carbonate.

20. A process according to claim 1, wherein the polymer inhibitor is hydroquinone or monomethyl ether hydroquinone.

21. A process according to claim 20, wherein the polymer inhibitor is p-methoxy phenol.

22. A process according to claim 1, wherein the acid in the step (d) is an inorganic or organic acid.

23. A process according to claim 22, wherein the acid is hydrochloride, sulfuric acid, acetic acid or oxalic acid.

24. A process according to claim 1, wherein the solvent in the step (d) is water, methanol, ethanol, acetonitrile, toluene, xylene, chlorobenzene, chloroform, dichloromethane or ethylene dichloride.

## Patentansprüche

1. Verfahren zur Herstellung von Triazolopyrimidinderivaten der Formel (I), 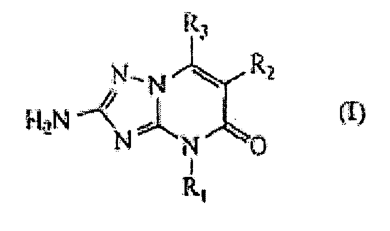 wobei
R₁ für einen Wasserstoff oder einen Alkylrest mit einem bis zehn Kohlenstoffatomen oder einen Cycloalkylrest mit drei bis sechs Kohlenstoffatomen, oder einen Alkenylrest mit bis zu vier Kohlenstoffatomen steht;
R₂ für einen Kohlenstoff, ein Halogenatom, einen Hydroxyalkyl- oder Alkylrest mit einem bis zehn Kohlenstoffatomen steht;
R₃ für einen Wasserstoff, einen Hydroxylalkyl- oder Alkylrest mit einem bis vier Kohlenstoffatomen steht;
das die folgenden Schritte umfasst
(a) Umsetzen des Dialkylcyanodithioiminocarbonats der Formel (V), wobei R für einen Alkylrest mit einem bis vier Kohlenstoffatomen steht, mit Alkylamin der Formel (IV), wobei R₁ die oben angegebene Bedeutung hat, in Gegenwart eines geeigneten Lösungsmittels bei Raumtemperatur, and danach tropfenweises Hinzufügen von Hydrazin nach Erhitzen bis zum Rückfluss, um eine Diamino-1,2,4-triazol-Verbindung der Formel (III) zu erhalten, wobei R₁ die oben angegebene Bedeutung hat;
(b) Umsetzen der Verbindung der Formel (III), wobei R₁ die oben angegebene Bedeutung hat, mit Aldehyd der Formel (VII), wobei R₈ für einen Alkylrest mit einem bis zehn Kohlenstoffatomen oder ein Phenyl oder ein substituiertes Phenyl steht, in Gegenwart eines organischen Lösungsmittels, um eine Iminverbindung der Formel (VI) zu ergeben, wobei R₁ und R₈ die oben angegebene Bedeutung haben, und wobei 1 bis 5 Äquivalent(e) von Aldehyd der Formel (VII) mit 1 Äquivalent des Diamino-1,2,4-triazols der Formel (III) bei 50~150 °C in einem organischen Lösungsmittel umgesetzt werden;
(c) Bilden von Imin der Formel (VIII), wobei R₁, R₂, R₃ und R₈ die oben angegebene Bedeutung haben, unter selektiver Kondensation von α,β-ungesättigten Säurederivaten der Formel (II), wobei R₂ und R₃ die oben angegebenen Bedeutungen haben, und R₅ für ein Halogenatom oder OR₉ steht, wobei R₉ für einen Alkylrest mit einem bis sechs Kohlenstoffatomen steht; und R₆ für einen Alkylrest mit einem bis sechs Kohlenstoffatomen steht, und eines Imins der Formel (VI), wobei R₁ und R₈ die oben angegebene Bedeutung haben, in Gegenwart einer Base und eines Polymerinhibitors in einem organischen Lösungsmittel bei Rückflusstemperatur; und
(d) Bilden von Triazolopyrimidinderivate der Formel (I) aus der Verbindung der Formel (VIII), wobei R₁, R₂, R₃ und R₈ die oben angegebenen Bedeutungen haben, in Gegenwart einer Säure bei Raumtemperatur bis Rückflusstemperatur.

2. Verfahren gemäß Anspruch 1, wobei in Schritt (a) das Alkylamin der Formel (IV) langsam der Mischung des Dialkylcyanodithioiminocarbonats der Formel (V) bei Raumtemperatur zugegeben wird und dann Hydrazin tropfenweise nach Erwärmen zum Rückfluss zugegeben wird, um eine Verbindung von Diamino-1,2,4-triazol der Formel (III) zu ergeben.

3. Verfahren gemäß Anspruch 1, wobei 1 bis 2 Äquivalente des Alkylamins verwendet werden.

4. Verfahren gemäß Anspruch 1, wobei 1 Äquivalent des Dialkylcyanodithioimino verwendet wird.

5. Verfahren gemäß Anspruch 1, wobei das Lösungsmittel in Schritt (a) Wasser, Acetonitril oder ein alkoholisches Lösungsmittel ist.

6. Verfahren gemäß Anspruch 5, wobei das alkoholische Lösungsmittel Methanol, Ethanol, Propanol oder Butanol ist.

7. Verfahren gemäß Anspruch 1, wobei 1 bis 4 Äquivalent(e) des Hydrazins verwendet werden.

8. Verfahren gemäß Anspruch 1, wobei Schritt (b) unter Säurekatalyse stattfindet.

9. Verfahren gemäß Anspruch 8, wobei der Säurekatalysator eine organische oder anorganische Säure ist.

10. Verfahren gemäß Anspruch 9, wobei der Säurekatalysator Essigsäure, Toluolsulfonsäure, Schwefelsäure oder Chlorwasserstoff ist.

11. Verfahren gemäß Anspruch 1, wobei das Lösungsmittel in Schritt (b) Acetonitril, Toluol, Xylol, Chlorbenzol, Chloroform, Dichlormethan, Ethylendichlorid, oder ein alkoholisches Lösungsmittel ist.

12. Verfahren gemäß Anspruch 11, wobei das alkoholische Lösungsmittel Methanol, Ethanol, Propanol oder Butanol ist.

13. Verfahren gemäß Anspruch 1, wobei in Schritt (c) 1 bis 2 Äquivalent(e) eines α,β-ungesättigten Säurederivates der Formel (II) mit 1 Äquivalent des Imins der Formel (VI) bei 40~150 °C umgesetzt werden.

14. Verfahren gemäß Anspruch 1, wobei 0,1 bis 2 Äquivalente der Base in Schritt (c) verwendet werden.

15. Verfahren gemäß Anspruch 1, wobei 0,005 bis 0,2 Äquivalente des Polymerinhibitors in Schritt (c) verwendet werden.

16. Verfahren gemäß Anspruch 1, wobei das Lösungsmittel in Schritt (c) Acetonitril, Toluol, Xylol, Chlorbenzol, Chloroform, Dichlormethan, oder Ethylendichlorid ist.

17. Verfahren gemäß Anspruch 1, wobei die Base in Schritt (c) ein Metallcarbonat oder Metallhydrogencarbonat ist.

18. Verfarhen gemäß Anspruch 17, wobei das Metall Li, Na, K, Rb, Cs, Be, Mg, Ca, Sr, oder Ba ist.

19. Verfahren gemäß Anspruch 17, wobei die Base Kaliumcarbonat ist.

20. Verfahren gemäß Anspruch 1, wobei der Polymerinhibitor Hydrochinon oder Monomethyletherhydrochinon ist.

21. Verfahren gemäß Anspruch 20, wobei der Polymerinhibitor p-Methoxyphenol ist.

22. Verfahren gemäß Anspruch 1, wobei die Säure in Schritt (d) eine anorganische oder organische Säure ist.

23. Verfahren gemäß Anspruch 22, wobei die Säure Chlorwasserstoff, Schwefelsäure, Essigsäure oder Oxalsäure ist.

24. Verfahren gemäß Anspruch 1, wobei das Lösungsmittel in Schritt (d) Wasser, Methanol, Ethanol, Acetonitril, Toluol, Xylol, Chlorbenzol, Chloroform, Dichlormethan, oder Ethylendichlorid ist.

## Revendications

1. Procédé pour la préparation de dérivés de la triazolo-pyrimidine de la formule (I), 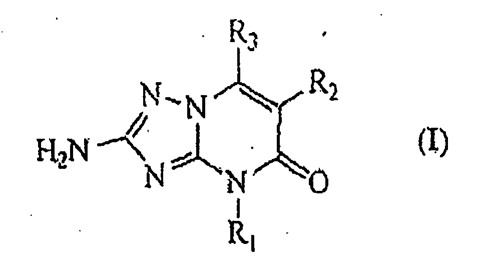 dans lequel
R₁ représente un radical hydrogène ou alkyle de un à dix atome(s) de carbone ou un radical cycloalkyle de trois à six atomes de carbone, ou un radical alkényle pouvant avoir jusqu'à quatre atomes de carbone ;
R₂ représente un hydrogène, un atome d'halogène, un radical hydroxyalkyle ou alkyle de un à dix atome(s) de carbone ;
R₃ représente un hydrogène, un radical hydroxyalkyle ou alkyle de un à quatre atome(s) de carbone ; comprenant les étapes consistant à
(a) faire réagir le carbonate de dialkyle cyanodithioimino de la formule (V), R₁ représentant un radical alkyle de un à quatre atome(s) de carbone, avec l'alkylamine de la formule (IV), R₁ ayant la signification indiquée ci-dessus, en présence d'un solvant approprié à température ambiante, puis à ajouter l'hydrazine goutte-à-goutte, après avoir chauffé jusqu'au reflux, pour obtenir un composé de diamino-1,2,4-triazole de la formule (III), R₁ ayant la signification indiquée ci-dessus ;
(b) faire réagir le composé de la formule (III), R₁ ayant la signification indiquée ci-dessus, avec l'aldéhyde de la formule (VII), R₈ représentant un radical alkyle de un à dix atome(s) de carbone, ou un phényle, ou un phényle substitué, en présence d'un solvant organique, pour obtenir un composé d'imine de la formule (VI), R₁ et R₈ ayant les significations indiquées plus haut, et dans lequel 1 à 5 équivalent(s) d'aldéhyde de la formule (VII) réagi(ssen)t avec 1 équivalent du diamino-1,2,4-triazole de la formule (III) dans un solvant organique à 50~150°C ;
(c) formation d'imine de la formule (VIII), R₁, R₂, R₃ et R₈ ayant les significations indiquées plus haut, avec une condensation sélective de dérivés d'acide α,β-insaturé de la formule (II), R₂ et R₃ ayant les significations indiquées plus haut, et R₅ représentant un atome d'halogène ou OR₉, R₉ représentant un radical alkyle de un à six atome(s) de carbone ; et R₆ représentant un radical alkyle de un à six atome(s) de carbone, et l'imine de la formule (VI), R₁ et R₈ ayant les significations indiquées plus haut, en présence d'une base et d'un inhibiteur de polymère dans un solvant organique à température de chauffage à reflux ; et
(d) formation de dérivés de triazolopyrimidine de la formule (I) du composé de la formule (VIII), R₁, R₂, R₃ et R₈ ayant les significations indiquées plus haut, en présence d'un acide à température ambiante jusqu'à la température de chauffage à reflux.

2. Procédé selon la revendication 1, dans lequel l'alkylamine de la formule (IV) est ajouté lentement au mélange de carbonate de dialkyle cyanodithioimino de la formule (V) à température ambiante, l'hydrazine étant ajoutée ensuite goutte-à-goutte après chauffage à reflux, pour obtenir un composé de diamino-1,2,4-triazole de la formule (III), à l'étape (a).

3. Procédé selon la revendication 1, dans lequel l'alkylamine est utilisé à 1 à 2 équivalent(s).

4. Procédé selon la revendication 1, dans lequel le dialkyle cyanodithioimino est utilisé à 1 équivalent.

5. Procédé selon la revendication 1, dans lequel le solvant de l'étape (a) est de l'eau, l'acétonitrile ou un solvant alcoolique.

6. Procédé selon la revendication 5, dans lequel le solvant alcoolique est du méthanol, de l'éthanol, du propanol ou du butanol.

7. Procédé selon la revendication 1, dans lequel l'hydrazine est utilisée à 1 à 4 équivalent(s).

8. Procédé selon la revendication 1, dans lequel l'étape (b) a lieu sous catalyse acide.

9. Procédé selon la revendication 8, dans lequel le catalyseur acide est un acide organique ou inorganique.

10. Procédé selon la revendication 9, dans lequel le catalyseur acide est de l'acide acétique, de l'acide toluènesulfonique, de l'acide sulfurique ou du chlorure d'hydrogène.

11. Procédé selon la revendication 1, dans lequel le solvant de l'étape (b) est l'acétonitrile, le toluène, le xylène, le chlorobenzène, le chloroforme, le dichlorométhane, le dichlorure d'éthylène ou un solvant alcoolique.

12. Procédé selon la revendication 11, dans lequel le solvant alcoolique est du méthanol, de l'éthanol, du propanol ou du butanol.

13. Procédé selon la revendication 1, dans lequel 1 à 2 équivalent(s) de dérivés d'acides α,β-insaturés de la formule (II) réagi(ssen)t avec 1 équivalent de l'imine de la formule (VI) à 40~150 °C à l'étape (c).

14. Procédé selon la revendication 1, dans lequel la base à l'étape (c) est utilisée à 0,1 à 2 équivalent(s).

15. Procédé selon la revendication 1, dans lequel l'inhibiteur de polymérisation à l'étape (c) est utilisé à 0,005 à 0,2 équivalent.

16. Procédé selon la revendication 1, dans lequel le solvant de l'étape (c) est l'acétonitrile, le toluène, le xylène, le chlorobenzène, le chloroforme, le dichlorométhane ou le dichlorure d'éthylène.

17. Procédé selon la revendication 1, dans lequel la base à l'étape (c) est un carbonate de métal ou un hydrogénocarbonate de métal.

18. Procédé selon la revendication 17, dans lequel le métal est Li, Na, K, Rb, Cs, Be, Mg, Ca, Sr ou Ba.

19. Procédé selon la revendication 17, dans lequel la base est du carbonate de potassium.

20. Procédé selon la revendication 1, dans lequel l'inhibiteur de polymérisation est l'hydroquinone ou le monométhyléther d'hydroquinone.

21. Procédé selon la revendication 20, dans lequel l'inhibiteur de polymérisation est le p-méthoxyphénol.

22. Procédé selon la revendication 1, dans lequel l'acide à l'étape (d) est un acide inorganique ou organique.

23. Procédé selon la revendication 22, dans lequel l'acide est de l'acide chlorhydrique, de l'acide sulfurique, de l'acide acétique ou de l'acide oxalique.

24. Procédé selon la revendication 1, dans lequel le solvant de l'étape (d) est de l'eau, du méthanol, de l'éthanol, de l'acétonitrile, du toluène, du xylène, du chlorobenzène, du chloroforme, du dichlorométhane ou du dichlorure d'éthylène.
